## Europäisches Patentamt

## European Patent Office

## Office européen des brevets

(11) Publication number: **0 193 112**
**A2**

(12) # EUROPEAN PATENT APPLICATION

(21) Application number: **86102206.9**

(22) Date of filing: **20.02.86**

(51) Int. Cl.⁴: **C 12 N 15/00**
**C 12 N 5/00, C 12 P 21/02**
**C 07 K 13/00**

(30) Priority: **22.02.85 US 704625**

(43) Date of publication of application:
**03.09.86 Bulletin 86/36**

(84) Designated Contracting States:
**AT BE CH DE FR GB IT LI LU NL SE**

(71) Applicant: **The Trustees of Columbia University in the City of New York**
**Broadway and West 116th Street**
**New York, NY 10027(US)**

(72) Inventor: **Soares, Marcelo Bento**
**100 Haven Avenue Tower No. 2 - Apt. 22B**
**New York New York 10032(US)**

(72) Inventor: **Efstratiadis, Argiris**
**426 Lydecker Street**
**Englewood New Jersey 07631(US)**

(74) Representative: **Henkel, Feiler, Hänzel & Partner**
**Möhlstrasse 37**
**D-8000 München 80(DE)**

(54) cDNA encoding mammalian insulin-like growth factor II.

(57) A cDNA molecule encoding a mammalian insulin-like growth factor II has been synthesized. These molecules or restriction enzyme fragments of these molecules can be inserted into suitable recombinant cloning vehicles and introduced into host cells. By culturing or growing these cells mammalian insulin-like growth factor II can be produced and isolated.

A specific embodiment of the invention concerns the synthesis of cDNA molecules encoding rat insulin-like growth factor 11, and production of polypeptide therefrom.

The invention also concerns cDNA molecules which correspond to mRNA transcripts encoding mammalian insulin-like growth factor II polypeptides. It has been discovered that these mRNA transcripts are predominantly expressed in specific tissues. The cDNA molecules may then be used to produce tissue-specific mammalian insulin-like growth factor II polypeptides.

The invention also provides probes and methods for the indentification and isolation of cDNA encoding mammalian insulin-like growth factor II polypeptides.

Croydon Printing Company Ltd.

cDNA ENCODING MAMMALIAN INSULIN-LIKE GROWTH FACTOR II

## Background of the Invention

This invention was made in part with Government support by a grant from the National Institute of Health, Department of Health and Human Services. The Government has certain rights in this invention.

Throughout this application various publications are referenced by numbers within parentheses. Full citations for these publications may be found at the end of the specification immediately preceding the claims. The disclosures of these publications in their entireties are hereby incorporated by reference into this application in order to more fully describe the state of the art to which this invention pertains.

The somatomedins (SMs) or insulin-like growth factors (IGFs), which have a structural resemblance to proinsulin, are mitogenic polypeptides that were thought to mediate the effects of the growth hormone (somatotropin) on skeletal tissue (see ref. 1-4 for reviews). Though this may be true for one of the two known SMs (IGF-I), the other (IGF-II), which is presumably involved in fetal development, seems to be under the influence of placental lactogen, but not of growth hormone (5,6).

The structures of human IGF-I (hIGF-I or SM-C) and IGF-II (hIGF-II or SM-A) have been determined (7-9). A rat basic SM, isolated from serum, has been partially sequenced (10), and it seems that it corresponds to hIGF-I. A second rat SM (previously designated "multiplication-stimulating activity" MSA), purified from serum- free medium conditioned by the Buffalo rat liver cell line BRL-3A (11), was shown by sequencing (12) to be the rat equivalent of hIGF-II (rIGF-II).

Peptide alignments with proinsulin (B chain-C peptide-A chain), show that the following regions can be defined in the sequences of the IGFs: Domains B, C and A. The IGFs also contain a fourth domain, D, which is not present in proinsulin (13). The limited amino acid sequence homology between the two types of molecules is restricted to the B and A domains. In contrast to insulin, which is generated from proinsulin by elimination of the middle C peptide, the C domain of the SMs remains in the active form of the molecules.

The structural similarities between proinsulin and IGFs suggested common ancestry of the corresponding genes (1). Thus, to expand our previous studies on the evolution (14-16) and expression (17) of insulin genes to other putative members of the insulin-like gene family, we cloned rIGF-II cDNA. This invention concerns the characterization of the mRNA species which encodes the protein precursor of rIGF-II. The invention also concerns transcripts that partially overlap the sequence of this message. We show that the family of these transcripts exhibits developmental and tissue specificity.

After completion of the experimental part of this work, two reports appeared describing the characterization of cDNA clones of the same sequence (18, 19). However, there are discrepancies between these reports and our data. The discrepancies concern the nucleotide sequence of various regions of the molecule and will be discussed in detail below.

## Summary of the Invention

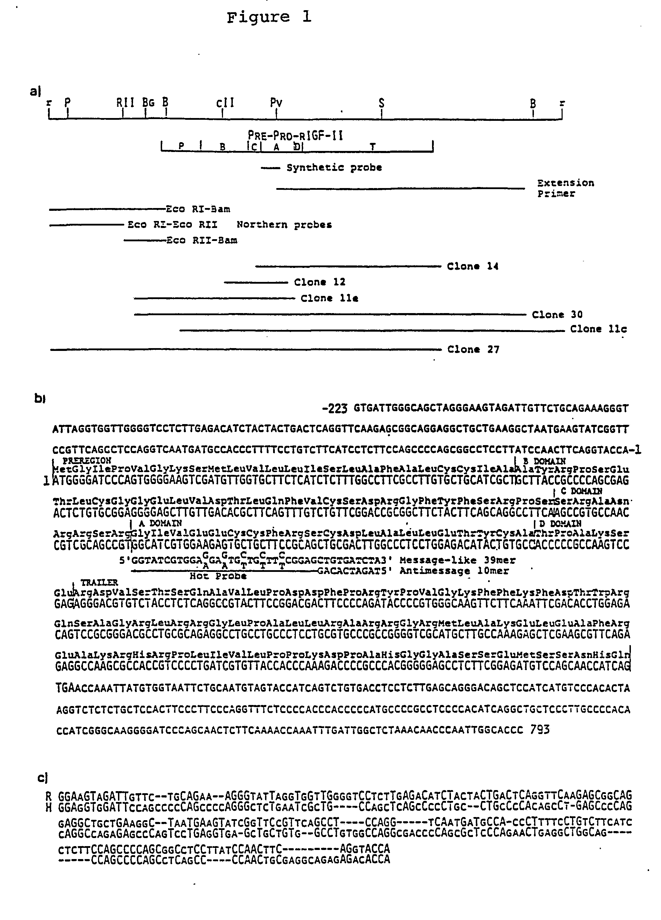

A cDNA molecule encoding mammalian insulin-like growth factor II has been discovered. A specific embodiment of the invention concerns a cDNA molecules encoding pre-pro rat insulin-like growth factor II. The nucleotide sequence of one such cDNA molecule encoding pre-pro-rIGF II is shown in Fig. 1 b. These molecules or restriction fragments of these DNA molecules may be inserted into recombinant cloning vehicles and then introduced into host cells.

The host cells containing the recombinant cloning vehicle with an inserted DNA sequence encoding mammalian IGF-II may be cultured or grown under suitable conditions permitting the expression of the DNA encoding mammalian IGF II. The mammalian IGF-II so produced can then be recovered using techniques known to those of ordinary skill in the art. The invention also concerns mammalian insulin-like growth factor polypeptides produced according to the methods of this invention.

cDNA molecules which correspond to different mRNA transcripts encoding mammalian insulin-like growth factor II polypeptides may also be produced. It has been discovered that some of these mRNA transcripts are predominantly expressed in specific tissues. These mRNA transcript molecules may exist as components of a heterologous population of a plurality mRNA molecules isolated from mammalian cells. cDNA molecules can be made corresponding to each of these mRNA transcripts. The cDNA of these transcripts can be inserted into a recombinant cloning vehicles and then into host cells. The host cells containing the recombinant cloning vehicles can be used according to the methods of this invention to produce tissue-specific mammalian insulin-like growth factor II polypeptides.

The invention also concerns a synthetic single-stranded DNA molecule encoding the first 13 amino acid residues of the A domain of rat insulin-like growth factor II. Single-stranded DNA molecules complementary to these DNA molecules along with restriction fragments of DNA molecules encoding pre-pro-rIGF-II may be labeled with detectable markers and used in methods for isolating cDNA molecules encoding other mammalian insulin-like growth factors IIs.

These methods of obtaining cDNA molecules encoding a mammalian insulin-like growth factor II comprise isolating poly $(A)^+$ mRNA from mammalian cells and using the RNA so isolated as a template for the synthesis of cDNA. The cDNA is cloned in a suitable recombinant cloning vehicle and in a suitable host cell in order to obtain a cDNA library. The cDNA library is then screened using one of the DNA molecules labeled with a detectable marker as a hybridization probe. The cDNA molecules that hybridize to the probe and therefore encode a growth factor II are identified and isolated.

## Brief Description of the Figures

Fig. 1: a) Restriction map of the pre-pro-rIGF-II cDNA sequence shown in b). Only some of the unique restriction sites (P=Pst, Bg=Bgl I, B=Bam, cII-Hinc II, Pv=Pvu II, and S-Sac I) are indicated. The Eco RII site (RII), used for the generation of hybridization probes, is not unique. The region encoding the rIGF-II precursor (P=preregion, domains B, C, A and D of rIGF-II, and T=trailer polypeptide) is indicated in a second line. The extent of the sequence carried by the characterized, overlapping cDNA clones is shown. The ends of all inserts are flanked by Eco RI linkers. The 5' linker of clone 27 and the 3' linker of clone IIc are indicated by 4 in the restriction map. b) Composite of the DNA sequence derived from the six clones shown in a). Small and large capital letters represent the noncoding (5' and 3') and coding regions, respectively. The position and sequence of the synthetic DNA oligonucleotides used for the generation of a labeled screening probe are shown below the corresponding sequence at the beginning of the A domain. c) Alignment of the 5' noncoding regions of rat (R) and human (H) (22) pre-pro-IGF-II cDNAs. Homologous regions are indicated by large capital letters. Gaps were introduced to maximize homology.

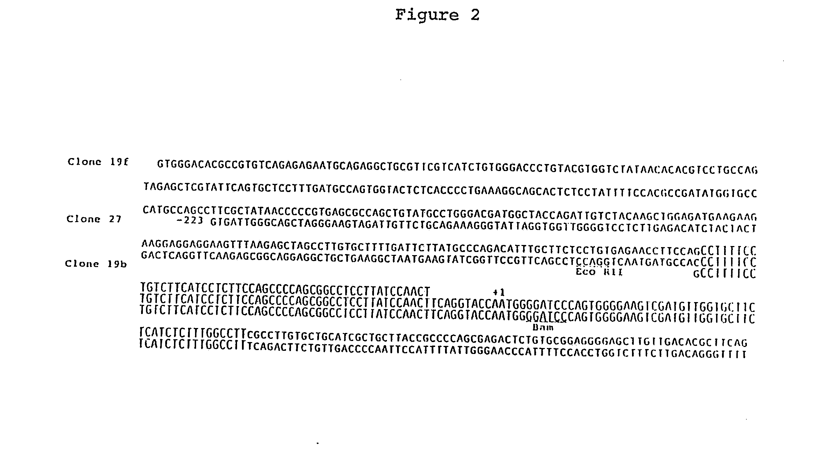

Fig. 2: Alignment of the sequences of clones 19f, 27 (pre-pro-rIGF-II) and 19b. Large capital letters indicate homologous sequences. For details see text. The insert of clone 19b (approximately 230 nt) has been only partially sequenced by the enzymatic method because of the presence of a very strong stop immediately downstream of the displayed sequence.

## Detailed Description of the Invention

A cDNA molecule encoding a mammalian insulin-like growth factor II polypeptide have been obtained. cDNA molecules can be synthesized which correspond to any of the mRNA transcript molecules which encode mammalian insulin-like growth factor II polypeptides.

Heterogeneous populations of mRNA transcripts which encode a mammalian insulin-like growth factor II polypeptide have been discovered. cDNA molecules may been synthesized from these mRNA transcripts, e.g. with reverse transcriptase. In certain embodiments of the invention the heterologous mRNA population consists of a plurality of mRNA molecules e.g. at least three mRNA transcripts. From these mRNA molecules a heterologous population of cDNA molecules encoding a mammalian insulin-like growth factor II can be produced.

For example in one embodiment of the invention a heterogeneous plurality of mRNA transcripts encoding rat insulin-like growth factor II polypeptides has been isolated. This population consists of at least three mRNA transcript molecules, three of which are about 3.4 kb, 1.75 kb and 1.6 kb in length each. Heterologous populations of cDNA molecules may be synthesized which correspond to these mRNA transcript molecules. It has also been discovered that these different mRNA transcripts may be predominantly expressed in specific tissues, e.g. the 1.6 kb and 1.75 kb rat IGF II mRNA transcripts in brain and lung tissue, and the 3.4 kb rat IGF II mRNA transcript in neonatal tissue and muscle tissue.

cDNA molecules encoding these different transcripts can be produced and inserted into recombinant cloning vehicles. The recombinant cloning vehicles containing these cDNAs could then be introduced into host cells in order to produce different tissue specific rIGF II polypeptide molecules.

Other mammalian cells e.g. bovine, procine, ovine, caprine human etc., may also contain such heterologous populations of a plurality of mRNA transcripts. The individual mRNA transcripts in these populations may also be predominantly expressed in specific tissues. cDNAs could then be made of the different mRNA transcripts and inserted into recombinant cloning vehicles. The recombinant cloning vehicles containing the different cDNA molecules could be introduced into host cells so that the cDNA could be expressed and different tissue-specific mammalian insulin-like growth factor II polypeptides could be produced and isolated. These tissue-specific mammalian growth factor II polypeptides could be useful in methods affecting the growth of mammals.

A cDNA molecule encoding pre-pro-rat insulin-like growth factor II has been obtained. This cDNA molecule has been restriction mapped and sequenced and these maps and sequences are depicted in Figure 1.

The cDNA molecules and restriction fragments of this invention may be inserted into recombinant cloning vehicles e.g. plasmids, cosmids or λ phage using methods known to those of ordinary skill in the art. The recombinant cloning vehicles can than be introduced into host cells, such as bacteria e.g. E. coli or eucaryotic cells, e.g. CHO cells, or yeast, using recombinant, transformation or other methods known to those skilled in the art.

These host cells containing the recombinant cloning vehicles with cDNA inserts encoding mammalian IGF II can be used in methods of producing mammalian IGF II polypeptides, e.g. rIGF II, pre-pro rIGF II. The methods comprise culturing or growing the host cells under suitable conditions known to those skilled in the art, which permit the expression of the DNA encoding the mammalian IGF II. The mammalian IGF II is then recovered from the cells or the surrounding medium using suitable methods known to those of ordinary skill in the art.

The invention also concerns the synthesis of a single-stranded DNA molecule encoding the first 13 amino acid residues of the A domain of rat insulin like growth factor II. The synthesis of this molecule is described below and the molecule itself is depicted in Figure 1b. A single-stranded DNA molecule complementary to this cDNA molecule has also been produced by using the cDNA molecule as a template for reverse transcriptase.

The cDNA molecule encoding the first 13 amino acids of the A domain of rIGF II, its complementary DNA molecule and DNA molecules which are restriction fragments of the cDNA molecule encoding pre-pro rIGF II may all be used as hybridization probes for cDNAs encoding other mammalian insulin-like growth factor II molecules e.g bovine, porcine, or ovine insulin-like growth factor II. These mammalian insulin-like growth factors may be useful in methods of controlling growth in mammals. To be used as probes these DNA molecules must be labeled with a detectable marker, e.g. radioactive, fluorescent and enzymatic markers. Methods of labeling DNA molecules are well known to those of ordinary skill in the art.

The methods of the invention for obtaining cDNA encoding a mammalian insulin-like growth factor II comprise isolating poly $(A)^+$ mRNA from mammalian cells using the standard procedures known to those of ordinary skill in the art. These mRNA molecules are then used as a template for the synthesis of cDNA, e.g. by reverse transcriptase. The cDNA is then cloned in a suitable cloning vehicle and in a suitable host cell in order to obtain a cDNA library, using standard recombinant DNA techniques such as those described below. The cDNA library so produced is then screened by using one of the DNA molecules labeled with a detectable marker and described above as a hybridization probe. The cDNA molecules that encode the mammalian insulin-like growth factor II and hybridize to the probe are then identified and isolated using the techniques described below.

## MATERIALS AND METHODS

### Materials

Restriction enzymes were from New England Biolabs or Bethesda Research Laboratories. T4 DNA ligase and Eco RI linkers were from New England Biolabs. Sl nuclease and T4 polynucleotide kinase were from Bethesda Research Laboratories. T4 DNA polymerase was from P-L Biochemicals. Klenow fragment of E. coli DNA polymerase was from Boehringer-Mannheim. Reverse transcriptase was from Life Sciences. Placental ribonuclease inhibitor (RNasin) was from Promega Biotec. Bacterial alkaline phosphatase was from Worthington. Nylon membranes (Gene Screen Plus), $\alpha$-$^{32}$P-dNTPs (800 Ci/mmole) and $\gamma$ -$^{32}$P-ATP (3000 Ci/mmole) were from New England Nuclear. DNA oligonucleotides were chemically synthesized using the Applied Biosystems 380A DNA Synthesizer. Bacteriophage vector $\lambda$ gtl0 and plasmid vector pUC9 were provided by T. Huynh and J. Messing, respectively. The BRL 3A cell line is widely available and can be obtained from the American Type Culture Collection, 12301 Parklawn Drive, Rockville, Md., 20852, where it is deposited under accession number ATCC CRL 1442.

RNA Purification

Cytoplasmic RNA was extracted as described (23) and further purified by oligo(dT)-cellulose chromatography. Total RNA was prepared by the guanidinium isothiocynate-CsCl procedure (24).

Recombinant DNA Procedures

Double-stranded cDNA was synthesized as described (23) using as a primer oligo(dT) and as a template cytoplasmic poly(A)$^+$ RNA from BRL-3A cells. The ends of the double-stranded cDNAs were made flush by treatment with T4 DNA polymerase, and the molecules were cloned as described (23) into the vector $\lambda$ gtl0, following at-tachment of Eco RI linkers. Screening of the library was performed as described (25).

The radioactive probe to isolate rIGF-II cDNA was made as follows: We first synthesized a message-like DNA 39mer, corresponding to the first 13 amino acid residues of the A domain of rIGF-II (see Fig. 1b). For the third position of the two-fold degenerate codons in the middle of the oligonucleotide we used both of the alternative bases, while for the rest of the codons the base of the third position was selected either at random or by giving advantage to possible G-U base-pairing. We then synthesized a 10mer, complementary to the 3' terminal region of the 39mer. Following annealing of the two oligonucleotides, the 10mer was extended with labeled triphosphates using the Klenow enzyme. It is our experience that this strategy is superior to the use of a mixture of shorter oligonucleotides containing all possible combinations. The success of this approach is due to the higher specific activity of the probe that can be obtained (in contrast to end-labeled oligonucleotides). Moreover, nucleation events in the middle of the probe (where an exact complement in relatively high representation is encountered) minimize the disadvantages of possible mismatches at the two ends.

A chromosomal DNA clone was isolated by screening with cloned cDNAs $6 \times 10^5$ plaques of a genomic rat DNA library (provided by T. Sargent). This library had been constructed by partial digestion of rat chromosomal DNA with Hae III, and the attachment of Eco RI linkers to the resulting fragments prior to ligation to λ Charon 4A arms.

## RNA and DNA Analyses

For Northern analysis, RNA was electrophoresed on formaldehyde-agarose gels and then transferred onto nylon membranes. Prehybridization and hybridization were as described (26), except when oligonucleotide probes were used (hybridization at 37°C, in 25% formamide). As probes we used labeled oligonucleotides (see above), nick-translated cloned restriction fragments or uniformly labeled single-strands synthesized on M13 templates by a modification of a published procedure (27). The inserts of the isolated cDNA clones were subcloned into M13mp9, M13mp10, or pUC9 for DNA sequencing, which was performed by the enzymatic method (28), using reverse transcriptase, or, in certain cases, by the chemical method (29), for verification.

RESULTS AND DISCUSSION

Isolation and Characterization of rIGF-II cDNA Clones

For the isolation of rIGF-II cDNA clones we first examined whether a radioactive antimessage probe, synthesized from a synthetic DNA template corresponding to the first 13 amino acid residues of the A domain of rIGF-II (see Materials and Methods and Fig. 1b), could hybridize to cytoplasmic poly(A)$^+$ RNA from the BRL-3A cell line. Northern analysis shows that an RNA species approximately 3.4 kb in length gave a strong hybridization signal.

Northern analysis of cytoplasmic poly(A)$^+$ RNA from BRL-3A cells was carried out as follows: The RNA (10 µ g per lane) was electrophoresed on a 1% agarose formaldehyde gel and then transferred onto a nylon membrane. The following probes were used: the labeled antimessage oligonucleotide (corresponding to the beginning of the A domain), synthesized on a synthetic DNA template (see Fig. 1a and b). A uniformly labeled single-stranded probe synthesized from a clone 11c (Fig. 1a) template, in M13. Because of the low concentration of the $\alpha$ -$^{32}$P-dNTPs during this reaction (which was not followed by chase), the synthesis was limited and the probe represented primarily 3' noncoding region

(see Fig. 1a). Sequential hybridization analysis was also performed. The following probes were used for sequential hybridization on the same membrane. A probe generated by nick-translation of the Eco RI-Eco RII fragment of clone 27 (see Fig. 1a). A uniformly labeled single-stranded probe sequence in Fig. 2 to position 274). The faint hybridization of the 3.4 kb of clone 19f homologous to this message (see Fig. 2). Labeled Hinf fragments of pBR322 were used as size markers. The slowest migrating band was determined to be a partial digestion product.

We then constructed a cDNA library of $1.5 \times 10^6$ recombinants from the BRL-3A RNA into the vector $\lambda$ gt10. Following library amplification, we screened $4.5 \times 10^5$ plaques with the same probe and identified 32 positive plaques, 25 of which were also positive upon rescreening. Sequencing of the insert of one of these clones (clone 14, Fig. 1a) identified a region of homology to the synthetic probe and a reading frame corresponding to the known amino acid sequence (12) of the A and D domains of rIGF-II.

Cross-hybridization of a clone 14 probe to the other 24 positive clones indicated that 11 of them carried rIGF-II sequence, while the rest were fortuitous hybridizers. Selected overlapping clones of the positive group (Fig. 1a) were also sequenced. Northern analysis of BRL-3A RNA using a 3' non-coding region probe from clone 11c showed that the same 3.4 kb RNA species hybridizing to the synthetic probe also hybridized to rIGF-II cDNA sequence.

A composite of our sequencing data is presented in Fig. 1b. Since the total length of the DNA sequence that we have established is 1016 nt, we are missing approximately 2.4 kb of the sequence of the 3.4 kb mRNA. A primer extension experiment suggested that the 5' end of the sequence is probably close to the 5' end of the 3.4 kb mRNA. As a primer for this extension we used a 536 nt long single-stranded antimessage DNA fragment (Fig. 1a), with a 3' end mapping at a Pvu II site (position +222 of the sequence). The longest abundant transcript we detected was approximately 1 kb in length. Assuming that this product does not correspond to a strong stop for reverse transcriptase, we calculate that the extended sequence (1000-536=464 nt) is only slightly longer than the distance between the Pvu II site and the 5' end of the sequence (444 nt). We note, however, that at least two other very faint bands of extension products, that can be seen only on the original autoradiogram, were longer than 1 kb. Their appearance might be due to rIGF-II-related sequences (see below). Thus, we tentatively conclude that most of the unknown sequence of the 3.4 kb mRNA corresponds to 3' noncoding region.

By identifying the rIGF-II residues in the sequence of Fig. 1b, and extending the translation frame upstream, we assigned the Met at amino acid position -24 as the initiator. We believe that this interpretation is correct because no other ATG triplets are present in the sequence between the -24 Met and the first upstream terminator (TGA at position -144) in the same reading frame. Moreover, the first 23 amino acid residues of this precursor, which presumably serve as a signal peptide for the secretory rIGF-II, contain a hydrophobic core, characteristic of all known preregions (30). Thus, the putative pre-pro-rIGF-II (179 residues) consists of a signal peptide (23 residues), the previously recognized (12) rIGF-II sequence (67 residues; B domain 32 residues, C domain 8residues, A domain 21 residues, D domain 6 residues), and a trailer polypeptide (89 residues), until the first terminator is encountered. This structure is the same at that of pre-pro-hIGF-II (18, 22), and analogous to that of pre-pro-hIGF-I (21), despite the dissimilarities between the two human factors in the sequences of their preregions and trailer polypeptides. Since the significance of the trailer sequence (if any) is unknown, it cannot be argued for the moment that the pre-pro-IGFs are yet another example of polyproteins (31) with more than one functional part.

The translated rIGF-II sequence is in agreement with the published protein sequence (12), except that the first residue of the C domain is a Ser (AGC), instead of a Gly (GGX). The assignment of a Gly in this position seems unequivocal from the protein sequencing data, while the fact that the nucleotide sequence is AGC (instead of GGC) has been firmly demonstrated by sequencing four independent clones. We feel that this excludes the possibility of reverse transcriptase errors. Thus, we do not know how to explain this difference, which is certainly not due to polymorphism, since the source of both the protein and the RNA was the same. We note that the residue in this position of the hIGF-II sequence (8, 18, 22) is also a Ser.

The calculated MW of the putative precursor is 19,968 daltons (preregion 2,330, rIGF-II 7,512, trailer 10,126). This size is consistent with a MW of 21 ± 1 K reported from the results of in vitro translation of fractionated BRL-3A RNA (32), which was designated as the "22 K" precursor. However, there is a significant discrepancy between the size of the 3.4 kb mRNA we detect and the reported size (12 S) of the translated fraction (32). Moreover, not all of the expected sizes of the processing products of pre-pro-rIGF-II, inferred from our sequencing data, correlate with the molecular species that have been observed in BRL-3A cells in vivo (33). From the translated DNA sequence, one would simply predict that the signal peptide is eliminated first, yielding a pro-protein of 17.6 K, which would then be processed to mature rIGF-II by clipping of the trailer polypeptide. However, the "22 K" in vitro precursor was not observed in vivo (33), even after a brief (10 min) $^{35}$S-Met pulse, while a 19-20 K species (thought to represent the pro-protein) was the first detectable product. Also, an intermediate species of 16 K, and two smaller forms of 8.7 and 7.1 K, were detected in vivo. The latter two species were also present in the culture media. Since the 7.1 K species is presumably the 7.5 K mature rIGF-II, a possible interpretation of these observations is that the trailer polypeptide is not separated from the body of rIGF-II in one step. This possibility is not unlikely because the trailer sequence contains one Lys-Arg and two Arg-Arg pairs, which are candidates for processing sites. If this scheme turns out to be correct, it would be of interest to examine the possible biological function of the small peptides that can be generated from the trailer. An alternative interpretation of these results (33) would be that the polyclonal antibody used in these studies recognizes also rIGF-II-related peptides.

The DNA sequencing data we present are only in partial agreement with the data by Dull et al. (18) and Whitfield et al. (19), who have also sequenced rIGF-II cDNA clones derived from the same mRNA source. Our data for the coding region of pre-pro-IGF-II are in complete agreement with the sequence of Dull et al. (18), including the first codon of the C domain (Ser instead of Gly). Thus, the accuracy of the sequence by Whitfield et al. (19), which has differences in six codons, should be re-examined. These authors presented the sequence of a partial cDNA clone, beginning with the last three codons of the C domain. They then extended this sequence upstream for another 35 codons by sequencing a primer-extended cDNA. Thus, their total sequence begins with the third residue of the B domain, and differs from ours in the third position of codons 3, 10, 22, 32 and 45, and in the first position of codon 33 (first codon of the C domain), which encodes Gly instead of Ser.

Our partial sequence of the 3' noncoding region covers 250 nt, and is in complete agreement with the longer sequence (392 nt) by Whitfield et al (19). The sequence by Dull et al. (18) is shorter in this region (134 nt). The last 16 nt of their sequence do not match our data, while the nucleotide at the fifth position upstream from this point is a T instead of a C (probably due to a misreading of the chemical sequencing ladder because of the presence of an Eco RII site).

The major discrepancy between our data and the sequence by Dull et al. (18) concerns the 5' noncoding region. With the exception of 9 nt upstream from the ATG initiator (where these authors position an intron in the corresponding sequence of the hIGF-II chromosomal gene), our sequence differs completely from theirs. The 5' noncoding region they present, derived from only one clone (pMSA-52H9), is 1055 nt long, which is inconsistent with the interpretation of our primer extension data. Moreover, an analogous primer extension
experiment by Whitfield et al. (19) yielded a size
estimate for this region, which is much closer to ours.
We believe that our sequence for the 5' noncoding region of the pre-pro-rIGF-II mRNA is correct for the
following reasons: a) Northern analysis indicates
that this sequence is present in the 3.4 kb mRNA. b)
The sequence reported by Bell et al. (22) as the 5'
noncoding region of hIGF-II cDNA cannot be found in the
hIGF-II gene sequence presented by Dull et al. (18),
even though it can be aligned to our rIGF-II 5'
noncoding region sequence (Fig. 1c), despite the expected considerable degree of divergence.

Transcripts Related to Pre-Pro-rIGF-II

Since our sequence for the 5' noncoding region of the
pre-pro-rIGF-II mRNA was derived exclusively from clone
27 (Fig. 1a), it was important to show that it did not
correspond to a cloning artifact, but to a region of
the 3.4 kb species. For this reason, we used as a
probe a nick-translated DNA fragment from clone 27,
extending from the extreme 5' end of the available
sequence (Eco RI linker used for cloning) to an Eco RII
site at position -79 (145 nt representing exclusively
5' noncoding region, Fig. 1a). Northern analysis of
BRL-3A RNA showed that this sequence is indeed part of
the 3.4 kb mRNA. Surprisingly, however, a second 1.6
kb RNA species was hybridized.

Thinking, after this result, that we might be dealing
with a case of differential splicing, we rescreened the
BRL-3A cDNA library with a 250 nt Eco RI-Bam probe
(Fig. 1a), which contains the entire available 5' non- coding region, and identified approximately 100 positive clones. Fifteen of these clones, selected at random, were first established as true-positives by rescreening with the same probe; they were then analyze by Southern blotting, using as probe clone 14 DNA, which carries rIGF-II coding region (Fig. la). Two of the clones (19f and 19b) which did not hybridized to the clone 14 probe, were sequenced (Fig. 2). Clone 19f (a partial cDNA) has an insert of 400 nt, of which the last 51 nt are absolutely homologous to nucleotides -11 to -61 of our pre-pro-rIGF-II sequence, while the sequence upstream from position -61 is totally different. The insert of clone 19b is shorter (approximately 230 nt). It is also partial sequence beginning only one nucleotide upstream from the breaking point of clones 19f and 27. This nucleotide is a G as in the clone 19f sequence, but this does not allow us to conclude that 19f and 19b are overlapping clones. The sequence of clone 19b is completely homologous to the pre-pro-rIGF-II sequence between -61 to +53 (which includes the first 18 codons of the preregion). The rest of the available sequence (73 nt) extending in the 3' direction diverges from the sequence of pre-pro-rIGF-II.

To examine whether the sequence of clone 19f was part of the 1.6 kb RNA, we made a probe from this clone that excluded the 19f/27 overlap, and analyzed by Northern blotting BRL-3A RNA. The result was unexpected, because instead of the 1.6 kb RNA, another RNA species of 1.75 kb was hybridized. However, when the 250 nt Eco RI-Bam probe from clone 27 was used as a probe, which includes the overlap of clones 19f, 19b and 27, not only the previously identified three RNA species (3.4, 1.75 and 1.6 kb) were hybridized, but also a fourth species of 1.1 kb gave a positive signal. Hybridization to the Eco RII-Bam fragment of clone 27 shows that only three of the RNAs (3.4, 1.75 and 1.1 kb) share this sequence, because the 1.6 kb species did not hybridize. Of the four related RNAs we detected, three (3.4, 1.75 and 1.6 kb) are polyadenylated species. Thus, in addition to the 3.4 kb pre-pro-rIGF-II mRNA, the 1.75 and 1.6 kb RNAs almost certainly also encode polypeptides. This cannot be claimed for the moment for the 1.1 kb species, because though its presence was detected in total cell RNA, we have not yet probed poly(A)$^+$ RNA for this transcript.

Northern analysis was performed on gel blots of total RNA (50 μg per lane) extracted from neonatal rat tissues of brain, heart, liver, lung, and muscle, or from adult tissues adult liver, and adult muscle or from BRL-3A cells. The probe was uniformly labeled antimessage single-stranded DNA corresponding to the Eco RI-Bam fragment of clone 27 (see Fig. 1a). Two aliquots (50 μg each) of total BRL-3A cell RNA were electrophoresed in parallel and hybridized after transfer either to a uniformly labeled probe corresponding to the Eco RII-Bam fragment of clone 27 (Fig. 1a) or to a nick-translated probe of the entire chromosomal clone hybridizing to rIGF-II sequences (see Materials and Methods and text). Another experiment concerned the electrophoresis and hybridization to 8 μg of poly(A)$^+$ RNA from BRL cells and 50 μg (each) of total RNA from mouse fetal liver or adult liver. The probe was derived from clone 27 by limited synthesis and represented primarily trailer polypeptide sequence.

In order to verify that the four related RNA species are not present exclusively in the transformed BRL-3A cell line, and in order to examine their possible developmental and tissue specificity, we hybridized total RNA from rat neonatal (day 2 rat) tissues to the Eco RI-Bam probe of clone 27. All of the neonatal (day 2 rat) tissues that were examined (brain, heart, liver, lung and muscle) contain all four transcripts, but in different amounts. (The neonatal muscle contains in addition a hybridizing band of 1.85 kb that appears unique to this tissue). In contrast, the adult liver and muscle tissues contain only RNA in the region of the 1.75/1.6 kb doublet (the two RNAs did not resolve well in this experiment). The developmental specificity of the 3.4 kb mRNA seems to be the same in the mouse, because a cross-hybridizing 3.4 kb transcript is present in fetal, but not in adult, mouse liver RNA. The absence of the 3.4 kb mRNA from the adult tissues is consistent with previous data supporting the notion that IGF-II is a fetal somatomedin (see ref. 6 and other ref. therein).

The differential expression of these transcripts in neonatal tissues is intriguing. Obviously muscle is the tissue with the highest concentration of the 3.4 kb and 1.1 kb species, while the 1.75 kb and 1.6 kb RNAs are under represented. The latter two species, however, predominate in brain and lung. It is interesting that the cardiac muscle has a profile distinct from that of skeletal muscle. It is also interesting that the hepatic BRL-3A cell line retains the profile of the four species as it appears in liver tissue.

What is the source of these related RNA species? The available data do not allow us to discriminate between expression of different genes sharing exons and differential splicing. (However, differential polyadenylation seems to be excluded from the data). Preliminary Southern analysis of rat chromosomal DNA yielded results compatible with the presence of a unique gene, when coding region of rIGF-II was used as a probe. However, hybridization to the 250 nt Eco RI-Bam probe of clone 27 complicated the picture, because of the appearance of additional bands, which cannot be accounted for. Nevertheless, the identity of the overlapping sequence between clones 27 and 19f (or 27 and 19b) cannot be easily explained by the presence of two different genes, because at least some minor degree of divergence should have been evident. This issue (more than one gene vs. differential splicing, or both) will be resolved from the characterization of a corresponding chromosomal gene that we have isolated. Though it is still unknown whether the representation of the 3.4 kb mRNA on this clone is partial or complete, we note that a probe of the entire chromosomal clone (about 13.2 kb insert, consisting of two Eco RI fragments of 10 and 3.2 kb) hybridizes to all of the RNA species, with the exception of the 1.6 kb transcript, and in addition to a 3.0 kb RNA. This newly detected transcript might belong to the family or to a coordinately expressed, linked gene.

The detection of a family of related transcripts suggests that our data and the data by Dull et al. (18) might eventually be reconciled. In this regard we note that a probe from clone 27, which primarily represents trailer polypeptide sequence, hybridizes not only to the 3.4 kb mRNA, but also to a 4.3 kb transcript. However, despite the suggestive size of this transcript, we have no data indicating homology to the 5' noncoding region of Dull et al. (18). Nevertheless, we feel that differential splicing is the most likely explanation for the surprising (and unprecedented) observation by these authors that more then 1 kb of sequence, considered as 5' noncoding region, is 80% homologous between the human and rat IGF-II clones.

We note that both of these sequences contain long open reading frames, which have the potential to encode proline-rich peptides (with some striking, but limited, homologies to retroviral gag proteins). In addition, differential splicing is consistent with the presence of an open reading frame in the available 3' noncoding region of the pre-pro-rIGF-II sequence. Alternatively, the 3.4 kb species might simply belong to a category of mRNAs with exceedingly long 3' noncoding regions, like, for example, the message encoding acetylcholine receptor (34).

Considering the questions generated from these data, we feel that it is premature to derive conclusions about the possible relationship between the IGF, insulin and relaxin genes, though the structure of the coding regions and the position of introns in the three gene types are not inconsistent with the postulated divergent (rather than convergent) mode of evolution (1, 18). However, the most exciting prospect for the immediate future is the study of the developmental and tissue-specific expression of the structurally related transcripts we observe, especially if they are also functionally related, and the response of the corresponding gene(s) to hormonal stimuli. In addition, it will be interesting to eventually examine whether the genes encoding IGFs or their receptors, or both, correspond to oncogenes, by analogy to the PDGF/c-sis and EGF receptor/v-erb relationships (35-37).

0193112

References

1.  Blundell, T.L. and Humbel, R.E. (1980) Nature 287, 781-787.

2.  Smith, E.L. (1983) in Growth and Maturation Factors, Guroff, G. Ed., vol. 1, pp. 293-323, Wiley and Sons, New York.

3.  Herington, A.C., Cornell, H.J., and Kuffer, A.D. (1983) Int. J. Biochem. 15, 1201-1210.

4.  Preece, M.A. (1983) in Hormones in Blood, Gray, C.H. and James, V.H.T. Eds., vol 4, pp. 87-108, Academic Press, New York.

5.  Scheonle, E., Zapf, J., Humbel, R.E., and Froesch, E.R. (1982) Nature 296, 252-253.

6.  Adams, S.O., Nissley, S.P., Handwerger, S., and Rechler, M.M. (1983) Nature 302, 150-153.

7.  Rinderknecht, E. and Humbel, R.E. (1978) FEBS Lett. 89, 283-286.

8.  Rinderknecht, E. and Humbel, R.E. (1978) J. Biol. Chem. 253, 2769-2775.

9.  Spencer, E.M., Ross, M., and Smith, B. (1983) in Insulin-like Growth Factors/Somatomedins, Spencer, E.M. Ed., pp. 81-96, de Gruyter, New York.

10. Rubin, J.S., Mariz, I., Jacobs, J.W., Daughaday, W.H., and Bradshaw, R.A. (1982) Endocrinology 110, 734-740.

11. Dulak, N.C. and Shing, Y.W. (1976) J. Cell Physiol. 90, 127-138.

12. Marquardt, H., Todaro, G.J., Henderson, L.E., and Oroszlan, S. (1981) J. Biol. Chem. 256, 6859-6865.

13. Dayhoff, M.O. (1978) Atlas of Protein Sequence and Structure, vol. 5 Supp. 3, p. 151, Natl. Biomed. Res. Found., Washington, D.C.

14. Lomedico, P., Rosenthal, N., Efstratiadis, A., Gilbert, W., Kolodner, R., and Tizard, R. (1979) Cell 18, 545-558.

15. Perler, F., Efstratiadis, A., Lomedico, P., Gilbert, W., Kolodner, R., and Dodgson, J. (1980) Cell 20, 555-566.

16. Chan, S.J., Episkopou, V., Zeitlin, S., Karathanasis, S.K., MacKrell, A., Steiner, D.F., and Efstratiadis, A. (1984) Proc. Nat. Acad. Sci. USA 81, 5046-5050.

17. Episkopou, V., Murphy, A.J.M., and Efstratiadis, A. (1984) Proc. Nat. Acad. Sci. USA 81, 4657-4661.

18. Dull, T.J., Gray, A., Hayflick, J.S., and Ullrich, A. (1984) Nature 310, 777-781.

19. Whitfield, H.J., Bruni, C.B., Frunzio, R., Terrell, J.E., Nissley, S.P., and Rechler, M.M. (1984) Nature 312, 277-280.

20. Ullrich, A., Berman, C.H., Dull, T.J., Gray, A., and Lee, J.M. (1984) EMBO J. 3, 361-364.

21. Jansen, M., van Schaik, F.M.A., Ricker, A.T., Bullock, B., Woods, D.E., Gabbay, K.H., Nussbaum, A.L., Sussenbach, J.S., and Van den Brande, J.L. (1983) Nature 306, 609-611.

22. Bell, G.I., Marrywhether, J.P., Pescador-Sanchez, R., Stempein, M.M., Priestley, L., Scott, J., and Rall, L.B. (1984) Nature 310, 775-777.

23. Maniatis, T., Fitsch, E.F., and Sambrook, J. (1982) Molecular Cloning: A Laboratory Manual, Cold Spring Harbon Laboratory, Cold Spring Harbor, New York.

24. Chirgwin, J.M., Przybyla, A.E., MacDonald, R.J., and Rutter, W.J. (1979) Biochemistry 18, 5294-5299.

25. Benton, W.D. and Davis, R.W. (1977) Science 196, 180-182.

26. Zeitlin, S. and Efstratiadis, A. (1984) Cell 39, 589-602.

27. Hu, N. and Messing, J. (1982) Gene 17, 271-277.

28. Sanger, F., Nicklen, S., and Coulson, A.R. (1977) Proc. Nat. Acad. Sci. USA 74, 5463-5467.

29. Maxam, A.M. and Gilbert, W. (1980) Meth. Enzymol. 65, 499-560.

30. Steiner, D.F., Quinn, P.S., Chan, S.J., Marsh, J., and Tager, H.S. (1980) Ann. N.Y. Acad. Sci. 343, 1-16.

31. Douglass, J., Civelli, O., and Herbert, E. (1984) Ann. Rev. Biochem. 53, 665-715.

32. Aquaviva, A.M., Bruni, C.B., Nissley, S.P., and Rechler, M.M. (1982) Diabetes 31, 656-658.

33. Yang, Y.W.-H., Acquaviva, A.M., Bruni, C.B., Romanus, J.A., Nissley, S.P., and Rechler, M.M. (1983) in Insulin-like Growth Factors/Somatomedins, Spencer, E.M. Ed., pp. 603-610, deGruyter, New York.

34. Noda, M., Furutani, Y., Takahashi, H., Toyosato, M., Tanabe, T., Shimizu, S., Kikyotani, S., Kayano, T., Hirose, T., Inayama, S., and Numa, S. (1983) Nature 305, 818-823.

35. Doolittle, R.F., Hunkapiller, M.W., Hood, L.E., Devare, S.G., Robbins, K.C., Aaronson, S.A., and Antoniades, H.N. (1983) Science 221, 275-277.

36. Waterfield, M.D., Scrace, G.T., Whittle, N., Stroobant, P., Johnsson, A., Wasteson, A., Westermark, B., Hedlin, C.-H., Huang, J.S., and Deuel, T.F. (1983) Nature 304, 35-39.

37. Ullrich, A., Coussens, L., Hayflick, J.S., Dull, T.J., Gray, A., Tam. A.W., Lee, J., Yarden, Y., Libermann, T.A., Schlessinger, J., Downward, J., Mayes, E.L.V., Whittle, N., Waterfield, M.D., and Seeburg, P.H. (1984) Nature 309, 418-425.

What is claimed is:

1. A cDNA molecule encoding a mammalian insulin-like growth factor II polypeptide.

2. A cDNA molecule of claim 1 which corresponds to a mRNA transcript encoding a mammalian insulin-like growth factor II polypeptide.

3. A cDNA molecule of claim 2 wherein the mRNA transcript encodes a mammalian pre-pro insulin-like growth factor.

4. A cDNA molecule of claim 2 wherein the mRNA transcript is predominantly expressed in a specific tissue.

5. A cDNA molecule of claim 2 wherein the mRNA transcript encodes rat insulin-like growth factor II.

6. A cDNA molecule of claim 3 wherein the mRNA transcript encodes pre-pro rat insulin-like growth factor II.

7. A cDNA molecule of claim 6, wherein the nucleotide sequence is the sequence depicted in Figure 1b.

8. A DNA molecule comprising a restriction enzyme fragment of the DNA molecule of claim 1.

9. A cDNA molecule of claim 5, wherein the mRNA transcript is about 3.4 kb in length.

10. A cDNA molecule of claim 5, wherein the mRNA transcript is about 1.75 kb in length.

11. A cDNA molecule of claim 5, wherein the mRNA transcript is about 1.6 kb in length.

12. A heterogeneous population of cDNAs, each of which encodes a mammalian insulin-like growth factor II polypeptide.

13. A heterogeneous population of claim 12 wherein the cDNAs correspond to a plurality of mRNA transcripts encoding mammalian insulin-like growth factor II polypeptides.

14. A heterogeneous population of claim 13 wherein the plurality of mRNA transcripts encoding mammalian insulin-like growth factor II polypeptides comprises at least three mRNA transcripts.

15. A heterogeneous population of claim 14 wherein the plurality of mRNA transcripts encode rat insulin-like growth factor II polypeptides and comprises three transcripts which are about 3.4 kb, 1.75 kb and 1.6 kb in length.

16. A recombinant cloning vehicle which includes the DNA molecule of claim 1.

17. A recombinant cloning vehicle which includes the DNA molecule of claim 2.

18. A recombinant cloning vehicle which includes the DNA molecule of claim 3.

19. A recombinant cloning vehicle which includes the DNA molecule of claim 4.

20. A recombinant cloning vehicle which includes the DNA molecule of claim 7.

21. A host cell which contains the vehicle of claim 16.

22. A host cell which contains the vehicle of claim 17.

23. A host cell which contains the vehicle of claim 18.

24. A host cell which contains the vehicle of claim 19.

25. A host cell which contains the vehicle of claim 20.

26. A method of producing mammalian insulin-like growth factor II polypeptide which comprises culturing or growing a cell of claim 21 under suitable conditions permitting expression of the DNA encoding mammalian insulin-like growth factor II polypeptide and recovering the growth factor polypeptide so produced.

27. A method of producing mammalian insulin-like growth factor II polypeptide which comprises culturing or growing a cell of claim 22 under suitable conditions permitting expression of the DNA encoding mammalian insulin-like growth factor II polypeptide and recovering the growth factor polypeptide so produced.

28. A method of producing mammalian pre-pro insulin-like growth factor II polypeptide which comprises culturing or growing a cell of claim 23 under suitable conditions permitting expression of the DNA encoding mammalian pre-pro insulin-like growth factor II polypeptide and recovering the growth factor polypeptide so produced.

29. A method of producing a tissue-specific mammalian insulin-like growth factor II polypeptide which comprises culturing or growing a cell of claim 24 under suitable conditions permitting expression of the DNA encoding the tissue-specific mammalian insulin-like growth factor II polypeptide and recovering the growth factor polypeptide so produced.

30. A method of producing a pre-pro-rat insulin-like growth factor II which comprises culturing or growing a cell of claim 25 under suitable conditions permitting expression of the DNA encoding pre-pro rat insulin-like growth factor II and recovering the pre-pro rat insulin-like growth factor so produced.

31. Mammalian insulin-like growth factor II polypeptide produced by the method of claim 26.

32. Mammalian insulin-like growth factor II polypeptide produced by the method of claim 27.

33. Mammalian pre-pro insulin-like growth factor II polypeptide produced by the method of claim 28.

34. A tissue-specific mammalian insulin-like growth factor II polypeptide produced by the method of claim 29.

35. Pre-pro rat insulin-like growth factor II produced by the method of claim 30.

36. A synthetic single-stranded DNA molecule encoding the first 13 amino acid residues of the A domain of rat insulin-like growth factor II.

37. A single-stranded DNA molecule complementary to the DNA molecule of claim 36.

38. A DNA molecule of claim 8, labeled with a detectable marker.

39. A DNA molecule of claim 36, labeled with a detectable marker.

40. A DNA molecule of claim 37, labeled with a detectable marker.

41. A method of obtaining cDNA encoding a mammalian insulin-like growth factor II which comprises isolating poly (A)$^+$ mRNA from mammalian cells, using the mRNA so isolated as a template for the synthesis of cDNA, cloning the cDNA in a suitable recombinant cloning vehicle and in a suitable host cell in order to obtain a cDNA library, screening the cDNA library so produced with the DNA molecule of claim 38 as a hybridization probe, and identifying and isolating the cDNA molecule encoding growth factor which hybridizes to the probe.

42. A method of obtaining cDNA encoding a mammalian insulin-like growth factor II which comprises isolating poly (A)$^+$ mRNA from mammalian cells, using the mRNA so isolated as a template for the synthesis of cDNA, cloning the cDNA in a suitable recombinant cloning vehicle and in a suitable host cell in order to obtain a cDNA library, screening the cDNA library so produced with the DNA molecule of claim 39 as a hybridization probe, and identifying and isolating the cDNA molecule encoding growth factor which hybridizes to the probe.

43. A method of obtaining cDNA encoding a mammalian insulin-like growth factor II which comprises isolating poly $(A)^+$ mRNA from mammalian cells, using the mRNA so isolated as a template for the synthesis of cDNA, cloning the cDNA in a suitable recombinant cloning vehicle and in a suitable host cell in order to obtain a cDNA library, screening the cDNA library so produced with the DNA molecule of claim 40 as a hybridization probe, and identifying and isolating the cDNA molecule encoding growth factor which hybridizes to the probe.

a)

r P        RII Bg B        cII        Pv                S                B        r

PRE-PRO-rIGF-II
|   P   |   B   |C|   A   D|                T                |

—— Synthetic probe

_____ Extension Primer

_____—Eco RI-Bam
_____— Eco RI-Eco RII    Northern probes
_____—Eco RII-Bam

_____ Clone 14
_____ Clone 12
_____ Clone 11e
_____ Clone 30
_____ Clone 11c
_____ Clone 27

b)

                                              -223 GTGATTGGGCAGCTAGGGAAGTAGATTGTTCTGCAGAAAGGGT

ATTAGGTGGTTGGGGTCCTCTTGAGACATCTACTACTGACTCAGGTTCAAGAGCGGCAGGAGGCTGCTGAAGGCTAATGAAGTATCGGTT

CCGTTCAGGCTCCAGGTCAATGATGCCACCCTTTTCCTGTCTTCATCCTCTTCCAGCCCCAGCGGCCTCCTTATCCAACTTCAGGTACCA-1
|PREREGION                                                      |B DOMAIN
MetGlyIleProValGlyLysSerMetLeuValLeuLeuIleSerLeuAlaPheAlaLeuCysCysIleAlaAlaTyrArgProSerGlu
1 ATGGGGATCCCAGTGGGGAAGTCGATGTTGGTGCTTCTCATCTCTTTGGCCTTCGCCTTGTGCTGCATCGCTGCTTACCGCCCCAGCGAG
                                                                         |C DOMAIN
ThrLeuCysGlyGlyGluLeuValAspThrLeuGlnPheValCysSerAspArgGlyPheTyrPheSerArgProSerSerArgAlaAsn
ACTCTGTGCGGAGGGGAGCTTGTTGACACGCTTCAGTTTGTCTGTTCGGACCGCGGCTTCTACTTCAGCAGGCCTTCAAGCCGTGCCAAC
            |A DOMAIN                                           |D DOMAIN
ArgArgSerArgGlyIleValGluGluCysCysPheArgSerCysAspLeuAlaLeuLeuGluThrTyrCysAlaThrProAlaLysSer
CGTCGCAGCCGTGGCATCGTGGAAGAGTGCTGCTTCCGCAGCTGCGACTTGGCCCTCCTGGAGACATACTGTGCCACCCCCGCCAAGTCC
          5'GGTATCGTGGA^GGA^GG^TG^GTG^CTT^CGGAGCTGTGATCTA3' Message-like 39mer
                          A   A   T   T   T
                   Hot Probe     ———————————GACACTAGAT5' Antimessage 10mer
|TRAILER
GluArgAspValSerThrSerGlnAlaValLeuProAspAspPheProArgTyrProValGlyLysPhePheLysPheAspThrTrpArg
GAGAGGGACGTGTCTACCTCTCAGGCCGTACTTCCGGACGACTTCCCCAGATACCCCGTGGGCAAGTTCTTCAAATTCGACACCTGGAGA

GlnSerAlaGlyArgLeuArgArgGlyLeuProAlaLeuLeuArgAlaArgArgGlyArgMetLeuAlaLysGluLeuGluAlaPheArg
CAGTCCGCGGGACGCCTGCGCAGAGGCCTGCCTGCCCTCCTGCGTGCCCGCCGGGGTCGCATGCTTGCCAAAGAGCTCGAAGCGTTCAGA

GluAlaLysArgHisArgProLeuIleValLeuProProLysAspProAlaHisGlyGlyAlaSerSerGluMetSerSerAsnHisGln|
GAGGCCAAGCGCCACCGTCCCCTGATCGTGTTACCACCCAAAGACCCCGCCCACGGGGGAGCCTCTTCGGAGATGTCCAGCAACCATCAG

TGAACCAAATTATGTGGTAATTCTGCAATGTAGTACCATCAGTCTGTGACCTCCTCTTGAGCAGGGACAGCTCCATCATGTCCCACACTA

AGGTCTCTCTGCTCCACTTCCCTTCCCAGGTTTCTCCCCACCCACCCCCATGCCCCGCCTCCCCACATCAGGCTGCTCCCCTTGCCCCACA

CCATCGGGCAAGGGGATCCCAGCAACTCTTCAAAACCAAATTTGATTGGCTCTAAACAACCCAATTGGCACCC 793

c)

R GGAAGTAGATTGTTC--TGCAGAA--AGGGTATTAGGTGGTTGGGGTCCTCTTGAGACATCTACTACTGACTCAGGTTCAAGAGCGGCAG
H GGAGGTGGATTCCAGCCCCCAGCCCCAGGGGCTCTGAATCGCTG-----CCAGCTCAGCCCCCTGC--CTGCCCCACAGCCT-GAGCCCCAG

GAGGCTGCTGAAGGC--TAATGAAGTATCGGTTCCGTTCAGCCT----CCAGG-----TCAatGATGCCA-ccCTTTTCCTGTCTTCATC
CAGGCCAGAGAGCCCAGTCCTGAGGTGA-GCTGCTGTG--GCCTGTGGCCAGGCGACCCCAGCGCTCCCAGAACTGAGGCTGGCAG----

CTCTTCCAGCCCCAGCGGCCTCCTTATCCAACTTC---------AGGTACCA
-----CCAGCCCCAGCCTCAGCC-----CCAACTGCGAGGCAGAGAGACACCA

# Figure 2

Clone 19f    GTGGGACACGCCGTGTCAGAGAGAATGCAGAGGCTGCGTTCGTCATCTGTGGGACCCTGTACGTGGTCTATAACACACGTCCTGCCAG

TAGAGCTCGTATTCAGTGCTCCTTTGATGCCAGTGGTACTCTCACCCCTGAAAGGCAGCACTCTCCTATTTTCCACGCCGATATGGTGCC

CATGCCAGCCTTCGCTATAACCCCCGTGAGCGCCAGCTGTATGCCTGGGACGATGGCTACCAGATTGTCTACAAGCTGGAGATGAAGAAG
Clone 27      -223 GTGATTGGGCAGCTAGGGAAGTAGATTGTTCTGCAGAAAGGGTATTAGGTGGTTGGGGTCCTCTTGAGACATCTACTACT

AAGGAGGAGGAAGTTTAAGAGCTAGCCTTGTGCTTTTGATTCTTATGCCCAGACATTTGCTTCTCCTGTGAGAACCTTCCAGCCTTTTCC
GACTCAGGTTCAAGAGCGGCAGGAGGCTGCTGAAGGCTAATGAAGTATCGGTTCCGTTCAGCCTCCAGGTCAATGATGCCACCCTTTTCC
Clone 19b                                                                      Eco RII            GCCTTTTCC

TGTCTTCATCCTCTTCCAGCCCCAGCGGCCTCCTTATCCAACT                    +1
TGTCTTCATCCTCTTCCAGCCCCAGCGGCCTCCTTATCCAACTTCAGGTACCAATGGGGATCCCAGTGGGGAAGTCGATGTTGGTGCTTC
TGTCTTCATCCTCTTCCAGCCCCAGCGGCCTCCTTATCCAACTTCAGGTACCAATGGGGATCCCAGTGGGGAAGTCGATGTTGGTGCTTC
                                                                     Bam

TCATCTCTTTGGCCTTCGCCTTGTGCTGCATCGCTGCTTACCGCCCCAGCGAGACTCTGTGCGGAGGGGAGCTTGTTGACACGCTTCAG
TCATCTCTTTGGCCTTTCAGACTTCTGTTGACCCCAATTCCATTTTATTGGGAACCCATTTTCCACCTGGTCTTTCTTGACAGGGTTTT

0193112